# FASCICULE DE BREVET EUROPEEN

(11) **EP 2 364 972 B1**
(45) Date de publication et mention de la délivrance du brevet: **19.09.2012**
(21) Numéro de dépôt: 11290091.5
(22) Date de dépôt: 16.02.2011
(51) Int. Cl.: C07D 223/16, C07C 235/74, C07C 323/60

(54) **"Nouveau procédé de synthese de l'Ivabradine et de ses sels d'Addition"**
Neues Syntheseverfahren von Ivabradin und seinen addition Salzen
New method for synthesising ivabradine and its addition salts

(30) Priorité: 17.02.2010 FR 1000657
(43) Date de publication de la demande: 14.09.2011
(73) Titulaire: Les Laboratoires Servier, 92284 Suresnes Cedex (FR)
(72) Inventeur: Peglion, Jean-Louis, 78110 Le Vesinet (FR); Caignard, Pascal, 93800 Epinay-sur-Seine (FR)

(56) Documents cités:
- WO-A1-2005/110993
- WO-A2-2008/146308
- LAURA A MCALLISTER ET AL: "A Fluorous-Phase Pummerer Cyclative-Capture Strategy for the Synthesis of Nitrogen Heterocycles", ANGEWANDTE CHEMIE. INTERNATIONAL EDITION, WILEY VCH VERLAG, WEINHEIM, vol. 44, no. 3, 7 janvier 2005 (2005-01-07), pages 452-455, XP007918373, ISSN: 1433-7851, DOI: DOI:10.1002/ANIE.200461930 [extrait le 2004-12-29]

## Description

La présente invention concerne un procédé de synthèse de l'ivabradine de formule (I) : 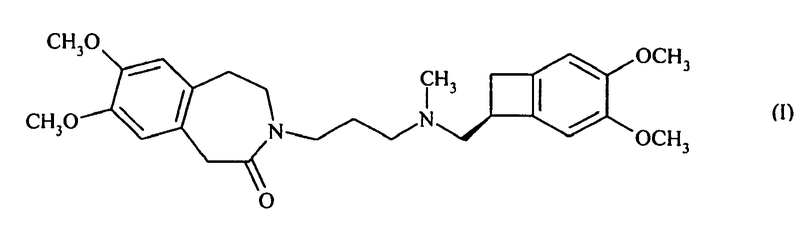 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H-*3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

WO 2008/146308 et WO 2005/110993 décrivent la synthèse d'ivabradine.

Ce brevet décrit la synthèse du chlorhydrate de l'ivabradine à partir du composé de formule (II) : 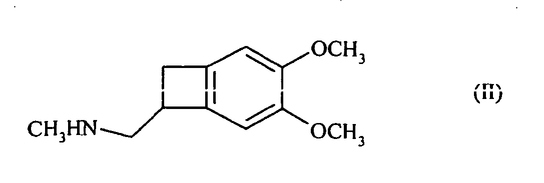 qui est dédoublé pour conduire au composé de formule (III) : 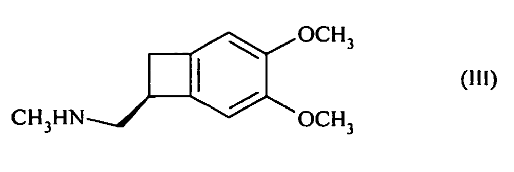 qui est mis en réaction avec le composé de formule (IV) : 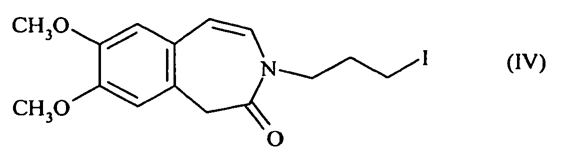 pour conduire au composé de formule (V) : 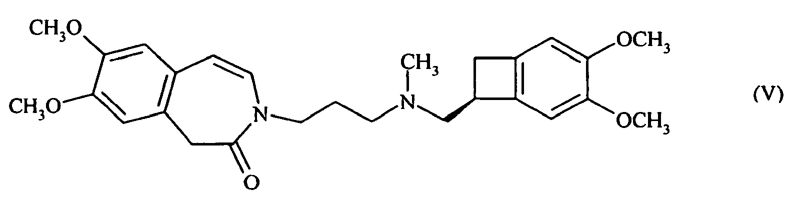 dont l'hydrogénation catalytique conduit à l'ivabradine, qui est alors transformée en son chlorhydrate.

L'inconvénient de cette voie de synthèse est de ne conduire à l'ivabradine qu'avec un rendement de 1%.

Compte-tenu de l'intérêt pharmaceutique de ce composé, il était important de pouvoir y accéder avec un procédé de synthèse performant, conduisant à l'ivabradine avec un bon rendement.

La présente invention concerne un procédé de synthèse de l'ivabradine de formule (1): 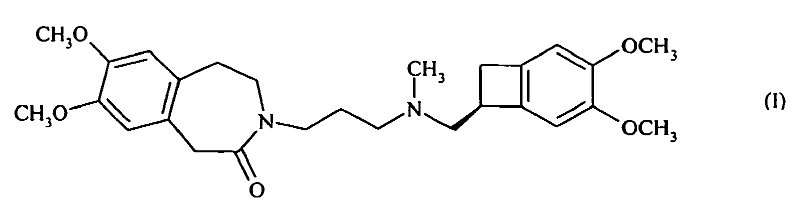 caractérisé en ce que l'on soumet le composé de formule (VI): 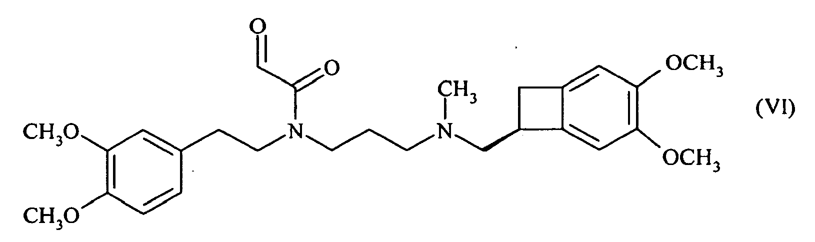 à l'action d'un thiol dans un solvant organique pour former l'hémithioacétal de formule (VII) : 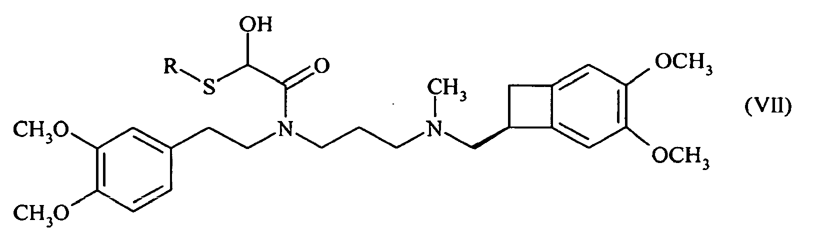 dans laquelle R représente un groupement alkyle linéaire ou ramifié, substitué ou non, optionnellement perfluoré, un groupement aryle substitué ou non, un groupement benzyle substitué ou non, ou un groupement CH₂CO₂Et,
lequel est soumis à une réaction de cyclisation pour conduire au composé de formule (VIII) : 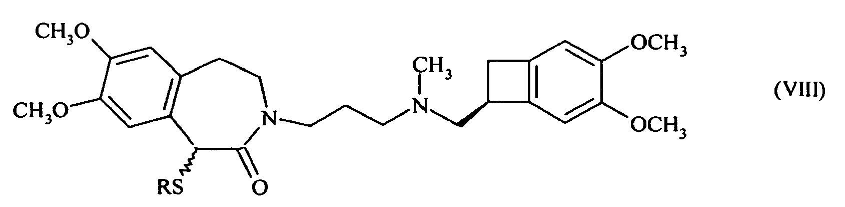 dans laquelle R est tel que défini précédemment,
lequel est soumis à une réaction de réduction pour conduire à l'ivabradine de formule (I), qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Le solvant préférentiellement utilisé dans la réaction de formation de l'hémithioacétal de formule (VII) est le dichlorométhane.

Le thiol préférentiellement choisi pour être mis en réaction avec le composé de formule (VI) est le thiophénol.

Le solvant préférentiellement utilisé pour la réaction de cyclisation du composé de formule (VII) en composé de formule (VIII) est le dichlorométhane.

Dans un mode de réalisation préféré de l'invention, la réaction de cyclisation du composé de formule (VII) en composé de formule (VIII) est effectuée en présence d'un réactif choisi parmi l'anhydride acétique, l'anhydride trifluoroacétique ou le trifluorométhanesulfonate de triméthylsilyle.

Plus préférentiellement, la réaction de cyclisation du composé de formule (VII) en composé de formule (VIII) est effectuée en présence d'anhydride trifluoroacétique.

Encore plus préférentiellement, la réaction de cyclisation du composé de formule (VII) en composé de formule (VIII) est effectuée en présence d'anhydride trifluoroacétique et d'un acide de Lewis choisi parmi BF₃.OEt₂, Sc(OTf)₃ ou Yb(OTf)₃.

Encore plus préférentiellement, la réaction de cyclisation du composé de formule (VII) en composé de formule (VIII) est effectuée en présence d'anhydride trifluoroacétique et de BF₃.OEt₂.

La réaction de réduction du composé de formule (VIII) est préférentiellement effectuée en présence de nickel de Raney dans l'éthanol ou en présence d'iodure de samarium(II) dans le tétrahydrofurane.

Les composés de formules (VI), (VII) et (VIII) sont des produits nouveaux, utiles comme intermédiaires de synthèse dans l'industrie chimique ou pharmaceutique, notamment dans la synthèse de l'ivabradine, de ses sels d'addition à un acide pharmaceutiquement acceptable et de leurs hydrates, et font à ce titre partie intégrante de la présente invention.

### Liste des abréviations utilisées :

DMF : *N,N-*diméthylformamide

DMSO : diméthylsulfoxyde

THF : tétrahydrofurane

IR : infrarouge

Les exemples ci-dessous illustrent l'invention.

Les points de fusion (PF) ont été mesurés au banc Köfler (BK).

Les spectres infrarouge ont été enregistrés sur un appareil Infra-rouge Bruker, tensor 27, accessoire ATR Golden Gate. Les produits sont déposés purs sur la platine.

### EXEMPLE 1 : [2-(3,4-diméthoxyphényl)éthyl]carbamate de tert-butyle

A une solution de 2-(3,4-diméthoxyphényl)éthylamine (10 g ; 55,2 mmol) dans le dichlorométhane (200 mL) est ajouté du dicarbonate de di-*tert*-butyle (12 g ; 55,2 mmol). Après 1 heure de contact à température ambiante, le milieu réactionnel est concentré sous pression réduite. Le résidu est repris dans le pentane (100 mL) et après 1 heure de contact à température ambiante, la suspension est filtrée sur fritté. 13,2 g de produit du titre sont obtenus sous forme d'un solide.

Rendement = 85%

PF = 65 ± 2°C.

### EXEMPLE 2 : 3-[{[(7S)-3,4-diméthoxybicyclo[4.2.0]octa-1,3,5-trién-7-yl]méthyl} (méthyl)amino]propyl[2-(3,4-diméthoxyphenyl)éthyl]carbamate de tert-butyle

A une solution du composé obtenu au stade précédent (7,6 g ; 27 mmol) dans 40 mL de DMF est ajouté par petites fractions à température ambiante du NaH (60 % dans l'huile) (1,14 g ; 28,5 mmol). Après 1 heure de contact à température ambiante, une solution de 3-chloro-*N-*{[(7S)-3,4-diméthoxybicyclo[4.2.0]octa-1,3,5-trién-7-yl]méthyl}-N-méthyl-1-propanamine (7,68 g ; 27 mmol) dans 16 mL de DMF est additionnée puis le milieu réactionnel est chauffé à 80°C pendant 3 heures. Après refroidissement à température ambiante, le milieu réactionnel est versé sur un mélange d'eau distillée et de glace. La phase aqueuse est extraite par l'acétate d'éthyle. Les phases organiques réunies sont séchées sur MgSO₄ puis concentrées sous pression réduite. Le résidu est purifié par chromatographie sur silice (CH₂Cl₂/EtOH : 95/5) et 9,1 g de produit du titre sont obtenus sous la forme d'une huile.

Rendement = 64%

IR : v = 3340, 1678, 1519, 1167 cm⁻¹ .

### EXEMPLE 3 : N-{[(7S)-3,4-diméthoxybicyclo[4.2.0]octa-1,3,5-trién-7-yl]méthyl}-N'-[2-(3,4-diméthoxyphényl)éthyl]-N-méthyl-1,3-propanediamine

9 g (17 mmol) du composé obtenu au stade précédent sont dissous dans une solution d'éthanol chlorhydrique à 2,8 N. Après 2 heures de contact à température ambiante, le milieu réactionnel est concentré sous pression réduite. Le résidu est repris dans de la soude 1N puis la phase aqueuse est extraite par l'acétate d'éthyle. Après séchage de la phase organique sur MgSO₄ puis concentration sous pression réduite, 6.8 g de produit du titre sont obtenus sous la forme d'une huile.

Rendement = 93%

IR : v = 3304, 2793, 1261, 1236, 1205, 1153 cm⁻¹.

### EXEMPLE 4 : N-{3-[{[(7S)-3,4-diméthoxybicyclo[4.2.0]octa-1,3,5-trién-7-yl]méthyl} (méthyl)amino]propyl}-N-[2-(3,4-diméthoxyphényl)éthyl]-2-hydroxyacétamide

### Stade 1 : 2-{13-[{[(7S)-3,4-diméthoxybicyclo[4.2.0]octa-1,3,5-trién-7-yl]méthyl) (méthyl)amino]propyl}[2-(3,4-diméthoxyphényl)éthyl]amino}-2-oxoéthyl acétate

A une solution du composé obtenu au stade précédent (6,8 g ; 15,8 mmol) dans 200 mL de dichlorométhane sont ajoutés à 0°C de la triéthylamine (3,1 g ; 22 mmol) puis goutte à goutte de l'acétoxyacétylchlorure (2,1 mL ; 19 mmol). Après 0,5 heure de contact à température ambiante, le milieu réactionnel est lavé par de l'eau distillée puis la phase organique est séchée sur MgSO₄. Après concentration sous pression réduite de la phase organique, 8 g de produit du titre sont obtenus sous la forme d'une huile et engagés sans purification au stade suivant.

### Stade2 : N-{3-[{[(7S)-3,4-diméthoxybicyclo[4.2.0]octa-1,3,5-trién-7-yl]méthyl}(méthyl) amino]propyl}-N-[2-(3,4-diméthoxyphényl)éthyl]-2-hydroxyacétamide

A une solution du composé obtenu au stade précédent (8 g) dans 80 mL d' un mélange eau/méthanol (2/1), est ajouté du K₂CO₃ (8,3 g ; 60,4 mmol). Après 1 heure de contact à température ambiante, le milieu réactionnel est concentré sous pression réduite puis le résidu est repris dans de l'eau distillée. Après extraction par l'acétate d'éthyle, les phases organiques jointes sont séchées sur MgSO₄ puis concentrées sous pression réduite. 6,2 g de produit du titre sont obtenus sous la forme d'une huile.

Rendement = 81 % (2 étapes)

IR : v = 3406, 2794, 1641, 1261, 1236, 1205, 1153 cm⁻¹

### EXEMPLE 5 : N-{3-[{[(7S)-3,4-diméthoxybicycto[4.2.0]octa-1,3,5-trién-7-yl]méthyl} (méthyl)amino]propyl)-N-[2-(3,4-diméthoxyphényl)éthyl]-2-oxoacétamide

A une solution de chlorure d'oxalyle (0,6 mL ; 6,77 mmol) dans 25 mL de dichlorométhane est additionnée à -78°C une solution de DMSO (0,9 mL ; 12,32 mmol) dans 5 mL de dichlorométhane. Après 1 heure de contact à -78°C, une solution du composé obtenu au stade précédent (3 g ; 6,16 mmol) dans 25 mL de dichlorométhane est ajoutée en 0,5 heure. Après 1 heure de contact à -78°C est additionnée de la triéthylamine (4,3 mL ; 30,8 mmol) puis le milieu réactionnel est agité 3 heures à température ambiante et ensuite versé sur une solution aqueuse saturée en NaHCO₃. La phase organique est séchée sur MgSO₄ puis concentrée sous pression réduite. 2,7 g de produit du titre sont obtenus sous la forme d'une huile.

Rendement = 89%

IR : v = 2788, 1645, 1589, 1261, 1236, 1207, 1151 cm⁻¹.

### EXEMPLE 6 : 3-{3-[{[(7S)-3,4-diméthoxybicyclo[4.2.0]octa-1,3,5-trién-7-yl]méthyl} (méthyl)amino]propyl}-7,8-diméthoxy-1-(phénylsulfanyl)-1,3,4,5-tétrahydro-2H-3-benzazépin-2-one

A une solution du composé obtenu au stade précédent (2,6 g ; 5,17 mmol) dans 60 mL de dichlorométhane est ajouté du thiophénol (0,53 mL ; 5,17 mmol). Après 1 nuit de contact à température ambiante, sont ajoutés successivement de l'anhydride trifluoroacétique (6,5 mL ; 47 mmol) puis BF₃.OEt₂ (6,5 mL ; 26 mmol). Le milieu réactionnel est agité 3 heures à température ambiante puis versé sur une solution aqueuse saturée en NaHCO₃. Le résidu obtenu après séchage sur MgSO₄ de la phase organique puis concentration sous pression réduite est purifié par chromatographie sur silice (CH₂Cl₂/EtOH/NH₄OH 28 % : 97/3/0,3). 1,15 g de produit du titre sont obtenus sous la forme d'une huile.

Rendement = 38%

IR : v =2790, 1641, 1245, 1205, 1174 cm⁻¹ .

### EXEMPLE 7 : 3-{3-[{[(7S)-3,4-diméthoxybicyclo[4.2.0)octa-1,3,5-trién-7-yl)méthyl} (méthyl)amino]propyl)-7,8-diméthoxy-1,3,4,5-tétrahydro-2H-3-benzazépin-2-one

A une solution du composé obtenu au stade précédent (0,8 g ; 1,39 mmol) dans l'éthanol est ajouté du Nickel de Raney (2,5 g) (50 % dans H₂O). Après 1 heure de contact au reflux, la suspension est refroidie puis filtrée sur Célite. 600 mg de produit du titre sont obtenus sous la forme d'une huile.

Rendement = 94%

IR : v = 2788, 1646, 1519, 1461, 1245, 1105 cm⁻¹.

### EXEMPLE 8 :chlorhydrate de 3-{3-[{[(7S)-3,4-diméthoxybicyclo[4.2.0)octa-1,3,5-trién-7-yl]méthyl}(méthyl)aminolpropyl}-7,8-diméthoxy-1,3,4,5-tétrahydro-2H-3-benzazépin-2-one

Le produit du titre est préparé à partir du produit obtenu au stade précédent en suivant le mode opératoire décrit dans le brevet EP 0 534 859 (Exemple 2, stade E).

## Revendications

1. Procédé de synthèse de l'ivabradine de formule (I): 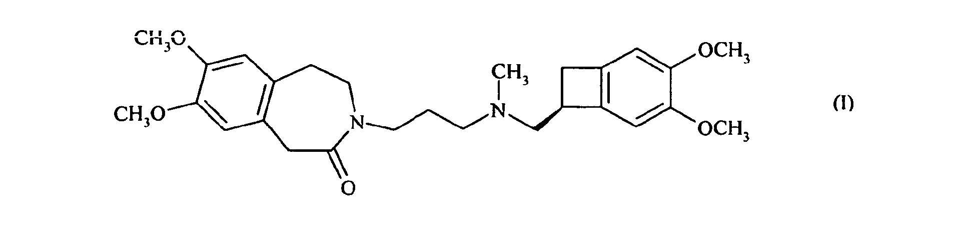 **caractérisé en ce que** l'on soumet le composé de formule (VI): 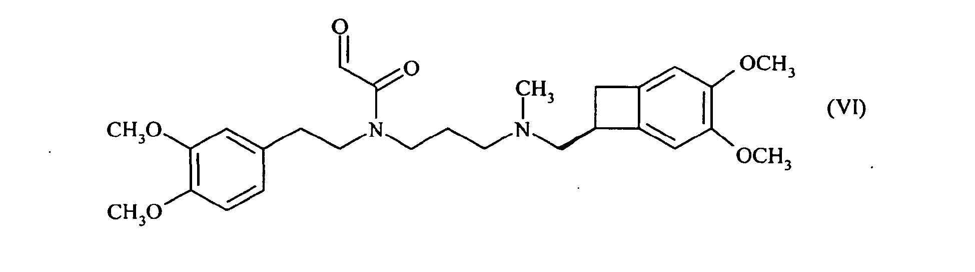 à l'action d'un thiol dans un solvant organique pour former l'hémithioacétal de formule (VII) : 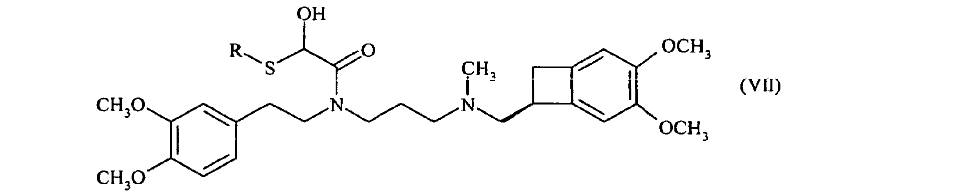 dans laquelle R représente un groupement alkyle linéaire ou ramifié, substitué ou non, optionnellement perfluoré, un groupement aryle substitué ou non, un groupement benzyle substitué ou non, ou un groupement CH₂CO₂Et,
lequel est soumis à une réaction de cyclisation pour conduire au composé de formule (VIII) : 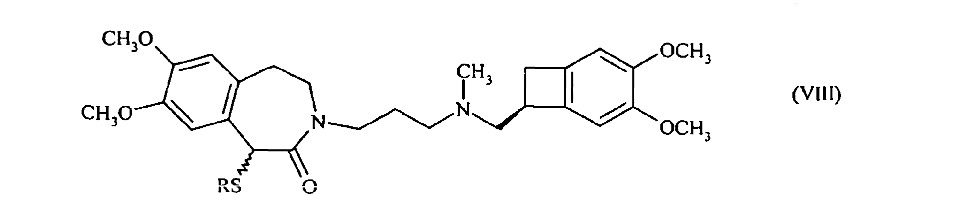 dans laquelle R est tel que défini précédemment,
lequel est soumis à une réaction de réduction pour conduire à l'ivabradine de formule (I), qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

2. Procédé de synthèse selon la revendication 1, **caractérisé en ce que** le solvant organique utilisé dans la réaction de formation de l'hémithioacétal de formule (VII) est le dichlorométhane.

3. Procédé de synthèse selon l'une des revendications 1 ou 2, **caractérisé en ce que** le thiol mis en réaction avec le composé de formule (VI) est le thiophénol.

4. Procédé de synthèse selon l'une des revendications 1 à 3, **caractérisé en ce que** le solvant utilisé dans la réaction de cyclisation du composé de formule (VII) en composé de formule (VIII) est le dichlorométhane.

5. Procédé de synthèse selon l'une des revendications 1 à 4, **caractérisé en ce que** la réaction de cyclisation du composé de formule (VII) en composé de formule (VIII) est effectuée en présence d'un réactif choisi parmi l'anhydride acétique, l'anhydride trifluoroacétique ou le trifluorométhanesulfonate de triméthylsilyle.

6. Procédé de synthèse selon la revendication 5, **caractérisé en ce que** la réaction de cyclisation du composé de formule (VII) en composé de formule (VIII) est effectuée en présence d'anhydride trifluoroacétique.

7. Procédé de synthèse selon la revendication 6, **caractérisé en ce que** la réaction de cyclisation du composé de formule (VII) en composé de formule (VIII) est effectuée en présence d'anhydride trifluoroacétique et d'un acide de Lewis choisi parmi BF₃.OEt₂, Sc(OTf)₃ ou Yb(OTf)₃.

8. Procédé de synthèse selon la revendication 7, **caractérisé en ce que** la réaction de cyclisation du composé de formule (VII) en composé de formule (VIII) est effectuée en présence d'anhydride trifluoroacétique et de BF₃.OEt₂.

9. Procédé de synthèse selon l'une des revendications 1 à 8, **caractérisé en ce que** la réaction de réduction du composé de formule (VIII) est effectuée en présence de nickel de Raney dans l'éthanol ou en présence d'iodure de samarium(II) dans le tétrahydrofurane.

10. Composé de formule (VI) :

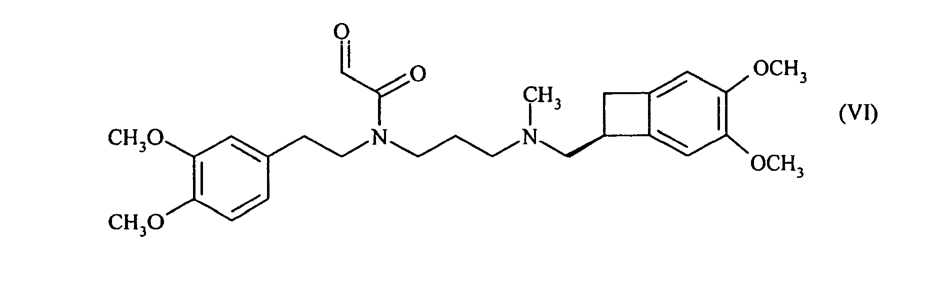

11. Composé de formule (VII) : 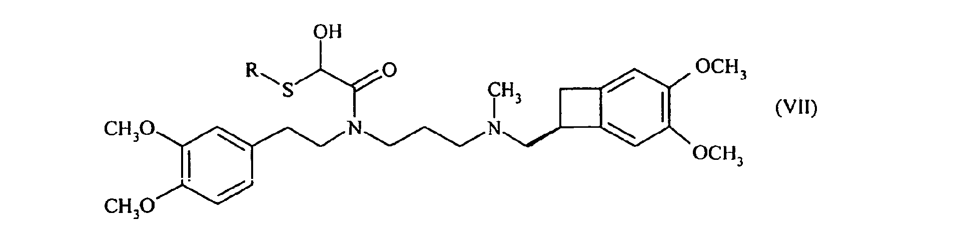 dans laquelle R représente un groupement alkyle linéaire ou ramifié, substitué ou non, optionnellement perfluoré, un groupement aryle substitué ou non, un groupement benzyle substitué ou non, ou un groupement CH₂CO₂Et.

12. Composé de formule (VIII) : 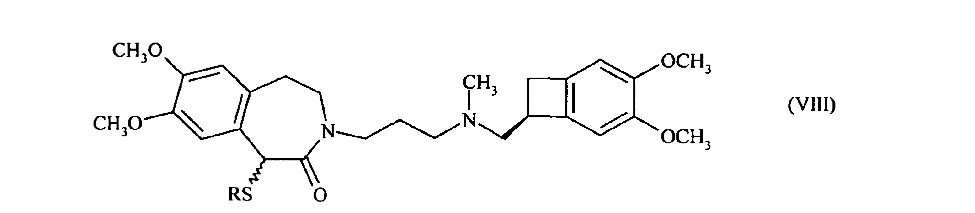 dans laquelle R est tel que défini à la revendication 11.

## Claims

1. Process for the synthesis of ivabradine of formula (I): 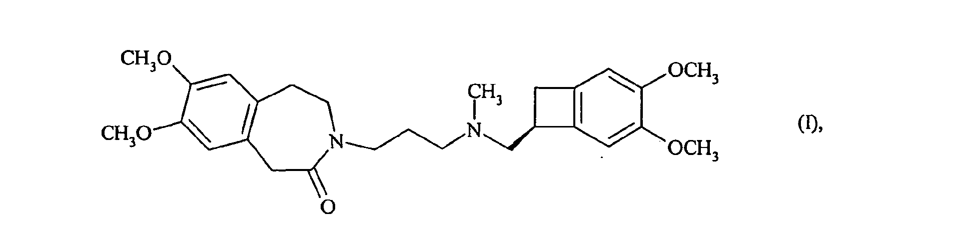 **characterised in that** the compound of formula (VI): 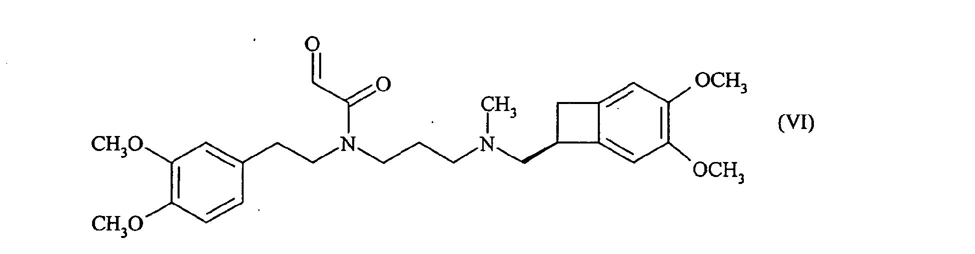 is subjected to the action of a thiol in an organic solvent to form the hemithioacetal of formula (VII): 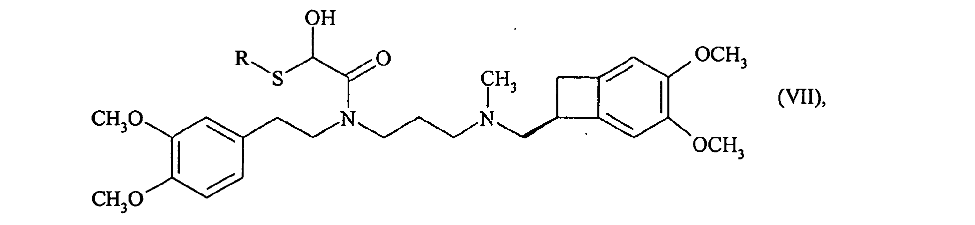 wherein R represents a substituted or unsubstituted, optionally perfluorinated, linear or branched alkyl group, a substituted or unsubstituted aryl group, a substituted or unsubstituted benzyl group or a group CH₂CO₂Et,
which is subjected to a cyclisation reaction to yield the compound of formula (VIII): 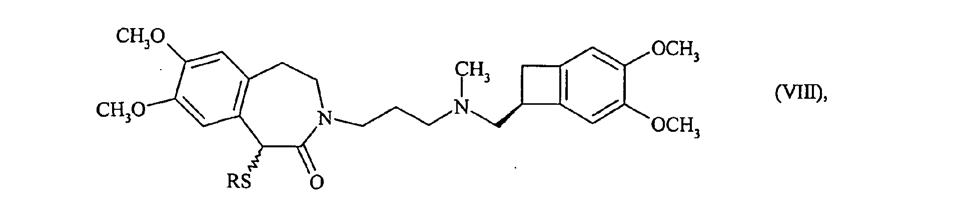 wherein R is as defined hereinbefore,
which is subjected to a reduction reaction to yield ivabradine of formula (I), which may optionally be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

2. Synthesis process according to claim 1, **characterised in that** the organic solvent used in the reaction for formation of the hemithioacetal of formula (VII) is dichloromethane.

3. Synthesis process according to either claim 1 or claim 2, **characterised in that** the thiol that is reacted with the compound of formula (VI) is thiophenol.

4. Synthesis process according to one of claims 1 to 3, **characterised in that** the solvent used in the reaction for cyclisation of the compound of formula (VII) to form the compound of formula (VIII) is dichloromethane.

5. Synthesis process according to one of claims 1 to 4, **characterised in that** the reaction for cyclisation of the compound of formula (VII) to form the compound of formula (VIII) is carried out in the presence of a reagent selected from acetic anhydride, trifluoroacetic anhydride and trimethylsilyl trifluoromethanesulphonate.

6. Synthesis process according to claim 5, **characterised in that** the reaction for cyclisation of the compound of formula (VII) to form the compound of formula (VIII) is carried out in the presence of trifluoroacetic anhydride.

7. Synthesis process according to claim 6, **characterised in that** the reaction for cyclisation of the compound of formula (VII) to form the compound of formula (VIII) is carried out in the presence of trifluoroacetic anhydride and a Lewis acid selected from BF₃.OEt₂, Sc(OTf)₃ and Yb(OTf)₃.

8. Synthesis process according to claim 7, **characterised in that** the reaction for cyclisation of the compound of formula (VII) to form the compound of formula (VIII) is carried out in the presence of trifluoroacetic anhydride and BF₃.OEt₂.

9. Synthesis process according to one of claims 1 to 8, **characterised in that** the reaction for reduction of the compound of formula (VIII) is carried out in the presence of Raney nickel in ethanol or in the presence of samarium(II) iodide in tetrahydrofuran.

10. Compound of formula (VI):

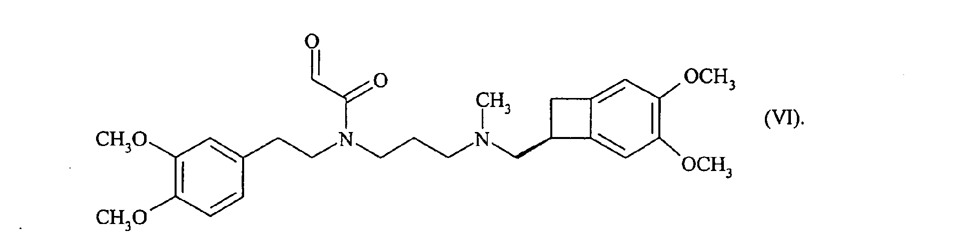

11. Compound of formula (VII): 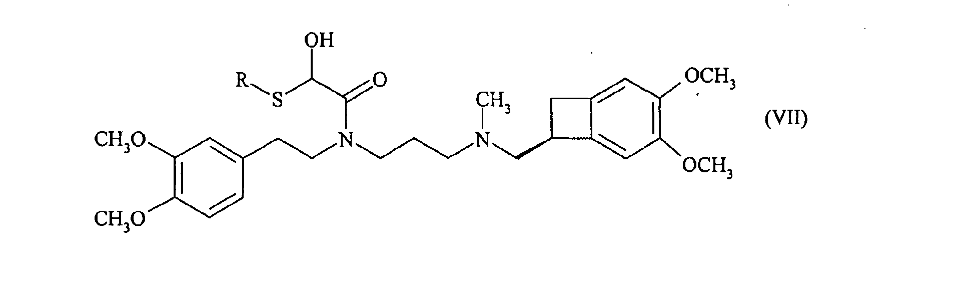 wherein R represents a substituted or unsubstituted, optionally perfluorinated, linear or branched alkyl group, a substituted or unsubstituted aryl group, a substituted or unsubstituted benzyl group or a group CH₂CO₂Et.

12. Compound of formula (VIII): 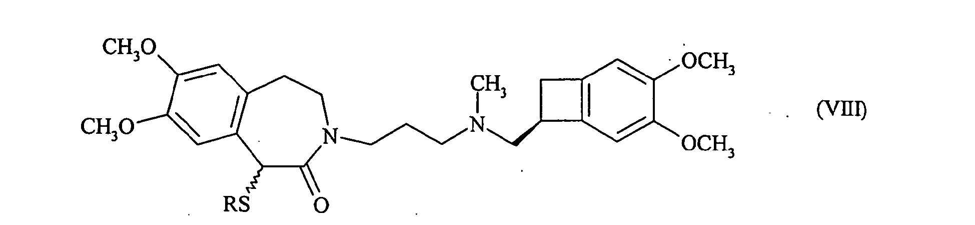 wherein R is as defined in claim 11.

## Patentansprüche

1. Verfahren zur Synthese von Ivabradin der Formel (I): 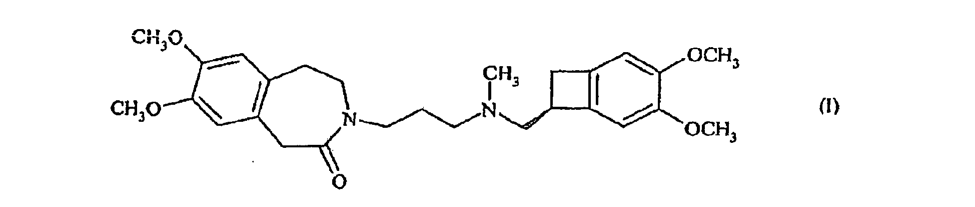 **dadurch gekennzeichnet, dass** man die Verbindung der Formel (VI): 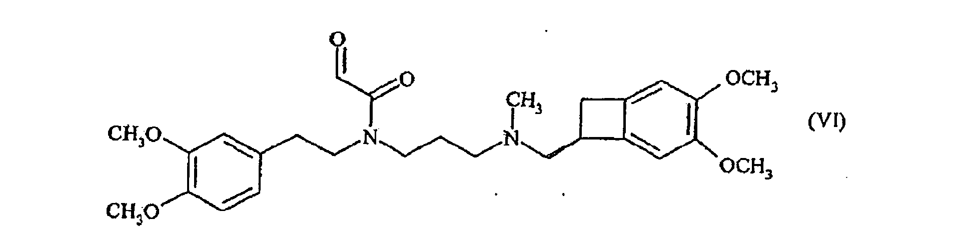 der Einwirkung eines Thiols in einem organischen Lösungsmittel unterwirft zur Bildung des Hemithioacetals der Formel (VII): 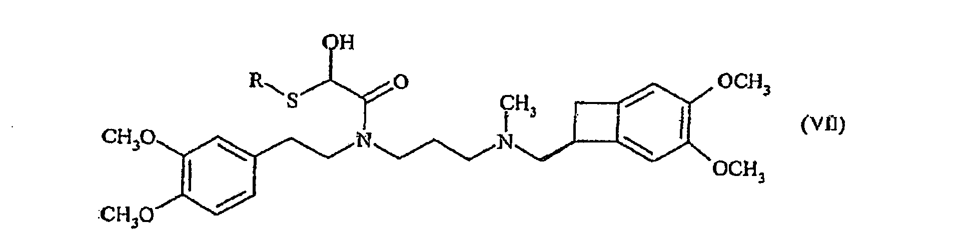 in der R eine geradkettige oder verzweigte, substituierte oder unsubstituierte, gegebenenfalls perfluorierte Alkylgruppe, gegebenenfalls substituierte Arylgruppe, gegebenenfalls substituierte Benzylgruppe oder eine Gruppe CH₂CO₂Et bedeutet,
welches man einer Cyclisierungsreaktion unterwirft zur Bildung der Verbindung der Formel (VIII): 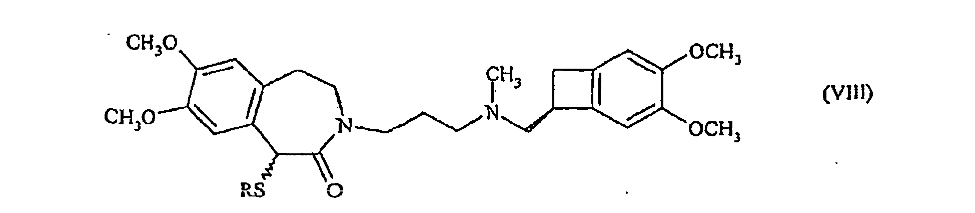 in der R die oben angegebenen Bedeutungen besitzt,
welche man einer Reduktionsreaktion unterwirft zur Bildung von Ivabradin der Formel (I), welches gegebenenfalls in seine Additionssalze mit einer pharmazeutisch annehmbaren Säure ausgewählt aus Chlorwasserstoffsäure, Bromwasserstoffsäure, Schwefelsäure, Phosphorsäure Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und in ihre Hydrate überführt werden kann.

2. Syntheseverfahren nach Anspruch 1, **dadurch gekennzeichnet, dass** das bei der Reaktion zur Bildung des Hemithioacetals der Formel (VII) verwendete organische Lösungsmittel Dichlormethan ist.

3. Syntheseverfahren nach einem der Ansprüche 1 oder 2, **dadurch gekennzeichnet, dass** das mit der Verbindung der Formel (VI) umgesetzte Thiol Thiophenol ist.

4. Syntheseverfahren nach einem der Ansprüche 1 bis 3, **dadurch gekennzeichnet, dass** das bei der Reaktion der Cyclisierung der Verbindung der Formel (VII) in die Verbindung der Formel (VIII) verwendete Lösungsmittel Dichlormethan ist.

5. Syntheseverfahren nach einem der Ansprüche 1 bis 4, **dadurch gekennzeichnet, dass** die Reaktion der Cyclisierung der Verbindung der Formel (VII) in die Verbindung der Formel (VIII) in Gegenwart eines Reagens durchgeführt wird, ausgewählt aus Essigsäureanhydrid, Trifluoressigsäureanhydrid oder Trimethylsilyltrifluormethansulfonat.

6. Syntheseverfahren nach Anspruch 5, **dadurch gekennzeichnet, dass** die Reaktion der Cyclisierung der Verbindung der Formel (VII) in die Verbindung der Formel (VIII) in Gegenwart von Trifluoressigsäureanhydrid durchgeführt wird.

7. Syntheseverfahren nach Anspruch 6, **dadurch gekennzeichnet, dass** die Reaktion der Cyclisierung der Verbindung der Formel (VII) in die Verbindung der Formel (VIII) in Gegenwart von Trifluoressigsäureanhydrid und einer Lewis-Säure ausgewählt aus BF₃·OEt₂, Sc(OTf)₃) oder Yb(OTf)₃ durchgeführt wird.

8. Syntheseverfahren nach Anspruch 7, **dadurch gekennzeichnet, dass** die Reaktion der Cyclisierung der Verbindung der Formel (VII) in die Verbindung der Formel (VIII) in Gegenwart von Trifluoressigsäureanhydrid und BF₃·OEt₂ durchgeführt wird.

9. Syntheseverfahren nach einem der Ansprüche 1 bis 8, **dadurch gekennzeichnet, dass** die Reaktion der Reduktion der Verbindung der Formel (VIII) in Gegenwart von Raney-Nickel in Ethanol oder in Gegenwart von Samarium(II)-iodid in Tetrahydrofuran durchgeführt wird.

10. Verbindung der Formel (VI):

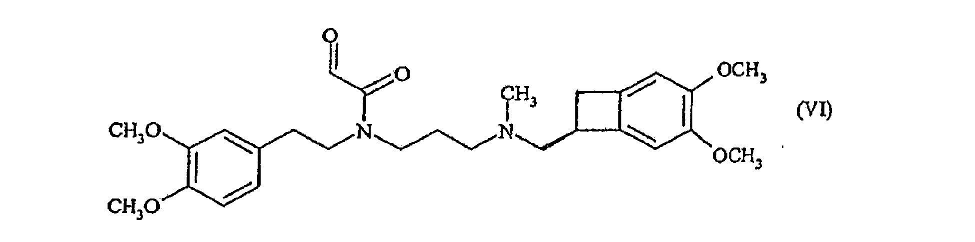

11. Verbindung der Formel (VII): 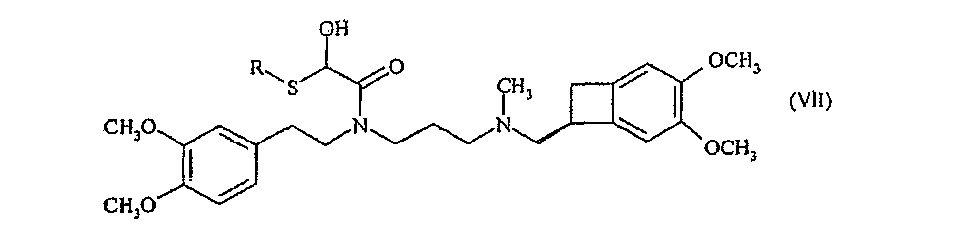 in der R eine geradkettige oder verzweigte, substituierte oder unsubstituierte, gegebenenfalls perfluorierte Alkylgruppe, eine substituierte oder nichtsubstituierte Arylgruppe, eine substituierte oder nichtsubstituierte Benzylgruppe oder eine Gruppe CH₂CO₂Et bedeutet.

12. Verbindung der Formel (VIII): 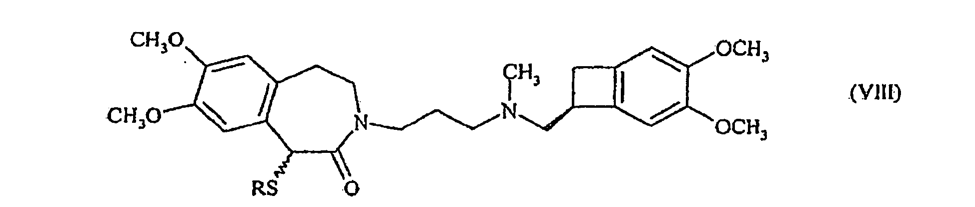 in der R die in Anspruch 11 angegebenen Bedeutungen besitzt.
